# EUROPEAN PATENT APPLICATION

(11) **EP 4 279 925 A2**
(43) Date of publication of application: **22.11.2023**
(21) Application number: 23188793.6
(22) Date of filing: 24.05.2018
(51) Int. Cl.: G01N 33/68

(54) **DULAGLUTIDE FOR THE TREATMENT OF CHRONIC KIDNEY DISEASE**

(30) Priority: 01.06.2017 US 201762513556 P
(62) Divisional of application: 18730908.3
(71) Applicant: Eli Lilly and Company, Indianapolis, IN 46285 (US)
(72) Inventor: BOTROS, Fady Talaat, Indianapolis, 46206-6288 (US); LAKSHMANAN, Mark Chandrakant, Indianapolis, 46206-6288 (US); TUTTLE, Katherine Rose, Indianapolis, 46206-6288 (US); ZIMMERMANN, Alan George, Indianapolis, 46206-6288 (US)
(74) Representative: Kent, Lindsey Ruth

(57) **Abstract**

The present invention relates to methods of using dulaglutide for the treatment of chronic kidney disease in patients having moderate to late stage chronic kidney disease.

## Description

The present invention relates to the field of medicine. More particularly, the present invention relates to methods for treating chronic kidney disease (CKD) comprising administering the GLP-1R agonist dulaglutide.

CKD is characterized by the progressive loss of kidney function, a major cause of which is diabetes mellitus. Diabetic nephropathy (DN) (also known as diabetic kidney disease) is one type of CKD, and is a chronic complication of diabetes mellitus. Increased albuminuria and gradual, progressive loss of renal function, measured as decline in estimated glomerular filtration rate (eGFR), are primary manifestations in human diabetic nephropathy. CKD patients experience over time a decrease in eGFR, and worsening CKD evolves into end stage renal disease (ESRD) for many patients, requiring either dialysis or kidney transplant, although once patients develop macroalbuminuria, the death rate may exceed the rate of progression to ESRD. Adler et al. (UKPDS 64) Kidney International, Vol. 63 (2003), pp. 225-232. eGFR is typically used to classify the severity of CKD for patients, with lower eGFR corresponding to more severe CKD. There are currently limited therapies that can modify the progression of existing kidney damage, so treatment options aimed at reducing the rate at which eGFR declines in patients are expected to delay or prevent the development of ESRD. Angiotensin converting enzyme (ACE) inhibitors or angiotensin receptor antagonists (ARBs) are used as current standard of care to slow the progression of CKD to ESRD, but these have been shown inadequate to prevent declines in kidney function or stop the ultimate progression to ESRD.

The glucagon like peptide 1 receptor (GLP-1R) agonist liraglutide has recently been described as reducing the progression of diabetic nephropathy over the course of a multiple year study, *see* J.F.Mann, et al., 27 J. Am. Soc. Nephrol. HI-ORD01 (2016); S.P. Marso, et al., NEJM (2016), doi:10.1056/NEJMoal1603827, and the once-weekly GLP-1R agonist semaglutide has been described as lowering rates of new or worsening nephropathy over the course of a multiple year study, see S.M. Marso, et al., NEJM (2016), doi:10.1056/NEJMoa1607141.

Nevertheless, there remains a need to provide alternative methods to treat patients with CKD stage 2 or worse, particularly methods which are capable of slowing the progression of the disease within as short a period of time as 26 weeks as compared to existing treatments, and which are capable of sustaining that attenuated progression, and even particularly in patients whose CKD has progressed to stage 3 or stage 4, or stage 2 with macroalbuminuria.

It has surprisingly been discovered that administration of dulaglutide according to the methods described herein is capable of meeting those needs. Dulaglutide is a GLP-1R agonist which has been sold under the tradename TRULICITY^{®} for the treatment of type 2 diabetes mellitus (T2DM) since 2014. Due to post-marketing reports of acute renal failure and worsening of chronic renal failure in patients treated with other GLP-1R agonists, the product insert for TRULICITY^{®} instructs healthcare providers to use caution when initiating or escalating doses and to monitor renal function in patients with renal impairment reporting severe adverse gastrointestinal reactions. *See* TRULICITY (dulaglutide) Highlights of Prescribing Information, Initial U.S. Approval: 2014, Section 5.5. The effects of dulaglutide on kidney function in T2DM patients having a broad range of kidney function was assessed in a recent publication which concluded that dulaglutide did not affect kidney function as measured by changes in eGFR. K.R. Tuttle, et al., Diabetes Obes. Metab. (2016), DOI: 10.1111/dom.12816. Despite the conclusions of that study, however, it has more recently been surprisingly discovered that, in certain CKD patients, not only does dulaglutide not have negative effects on kidney function, it actually significantly reduces the rate at which eGFR decreases. Moreover, such significant improvements in the rate of eGFR decrease may be seen in as short a period of time as 6 months after initiation of treatment with dulaglutide. The benefits of the methods of the present invention are many, and include the potential to significantly increase the amount of time before CKD patients reach ESRD or cardiovascular death, particularly in patients with more progressed CKD receiving maximal tolerated doses of ACE inhibitors or ARBs. Moreover, the methods of the present invention may also provide these CKD-related benefits independent of benefits related to glycemic control improvements.

Accordingly, the present invention provides a method of treating CKD in a patient, comprising: (a) identifying a patient having either: (i) eGFR between 15-59 mL/min/1.73 m²; or (ii) UACR ≥ 30 mg/g and kidney damage and eGFR between 60-89 mL/min/1.73 m²; (b) administering said patient an effective amount of dulaglutide once a week; and (c) continuing said once a week administration for at least 6 months; and wherein said administration results in attenuation in the progression of the patient's CKD.

In certain aspects, the present invention provides a method of decreasing the rate of loss of eGFR in a patient having CKD, comprising: (a) identifying a patient having either: (i) eGFR between 15-59 mL/min/1.73 m²; or (ii) UACR ≥ 30 mg/g and eGFR between 60-89 mL/min/1.73 m²; (b) administering said patient an effective amount of dulaglutide once a week; and (c) continuing said once a week administration for at least 6 months.

In another aspect, the present invention provides dulaglutide for use in treating CKD in a patient, wherein the patient has either: (i) eGFR between 15-59 mL/min/1.73 m²; or (ii) UACR ≥ 30 mg/g and eGFR between 60-89 mL/min/1.73 m²; and wherein an effective amount of dulaglutide is administered once a week for at least 6 months.

In another aspect, the present invention provides dulaglutide for use in decreasing the rate of loss of eGFR in a patient having CKD, wherein the patient has either: (i) eGFR between 15-59 mL/min/1.73 m²; or (ii) UACR ≥ 30 mg/g and eGFR between 60-89 mL/min/1.73 m²; and wherein an effective amount of dulaglutide is administered once a week for at least 6 months.

In certain embodiments the patient's CKD is not caused by T2DM. In certain embodiments the patient's CKD is not caused by diabetes. In certain embodiments the patient's CKD is caused by hypertension.

In other embodiments the patient's CKD is caused by diabetes. In certain embodiments the patient's CKD is caused by T2DM. In certain embodiments the patient's CKD is caused by T1DM.

In certain embodiments, the patient has eGFR between 15-59 mL/min/1.73 m². In certain embodiments, the patient has eGFR between 15-29 mL/min/1.73 m². In certain embodiments, the patient has eGFR between 30-59 mL/min/1.73 m². In certain embodiments, the patient has eGFR between 30-44 mL/min/1.73 m². In certain embodiments, the patient has eGFR between 45-59 mL/min/1.73 m². In certain embodiments, the patient has eGFR between 15-44 mL/min/1.73 m². In certain embodiments, the patient has eGFR between 60-75 mL/min/1.73 m².

In certain embodiments, the patient has a UACR between 30-300 mg/g. In certain embodiments, the patient has an UACR greater than 300 mg/g.

In certain embodiments, said administration is continued for at least 1 year. In other embodiments, said administration is continued for at least 2 years. In other embodiments, said administration is continued for at least 3 years. In other embodiments, said administration is continued for at least 4 years. In other embodiments, said administration is continued for at least 5 years. In other embodiments, said administration is continued for at least 10 years.

In certain embodiments, the effective amount of dulaglutide provided once a week is between 0.75-4.5 mg. In other embodiments, the effective amount of dulaglutide provided once a week is between 0.75 mg and 4.5 mg. In other embodiments, the effective amount of dulaglutide provided once a week is either 0.75 mg, 1.5 mg, 3.0 mg or 4.5 mg.

In certain embodiments, said attenuation in progression of the patient's CKD is reflected in an increase in time to one or more of: a 40% decrease in eGFR; progression to ESRD; renal death; and/or cardiovascular death.

In certain embodiments, said attenuation in progression of the patient's CKD is reflected in an increase in time to one or more of: a 50% decrease in eGFR; progression to ESRD; renal death; and/or cardiovascular death.

In certain embodiments, said attenuation in progression of the patient's CKD is reflected in an increase in time to a 40% decrease in eGFR.

In certain embodiments, said attenuation in progression of the patient's CKD is reflected in an increase in time to a 50% decrease in eGFR.

In certain embodiments, said attenuation in progression of the patient's CKD is reflected in an increase in time to progression to ESRD.

In certain embodiments, said attenuation in progression of the patient's CKD is reflected in an increase in time to renal death.

In certain embodiments, said attenuation in progression of the patient's CKD is reflected in an increase in time to cardiovascular death.

In certain embodiments, said attenuation in progression of the patient's CKD is reflected in a 15-30% reduced hazard ratio. In certain embodiments, said attenuation in progression of the patient's CKD is reflected in at least a 15% reduced hazard ratio. In certain embodiments, said attenuation in progression of the patient's CKD is reflected in at least a 20% reduced hazard ratio.

In certain embodiments, said attenuation in the progression of the patient's CKD is not entirely dependent on reductions in the patient's HbA1c.

In certain embodiments, dulaglutide is administered in simultaneous or sequential combination with an ACE inhibitor and/or an ARB. In certain embodiments, the ACE inhibitor is selected from the group consisting of zofenopril, perindopril, trandolapril, captopril, enalapril, lisinopril, and ramipril. In certain embodiments, the ARB is selected from the group consisting of valsartan, telmisartan, losartan, irbesartan, azilsartan, olmesartan, candesartan, eprosartan and fimasartan.

Dulaglutide is a human GLP-1R agonist which comprises a dimer of a GLP-1 analog fused at its C-terminus via a peptide linker to the N-terminus of an analog of an Fc portion of an immunoglobulin, and is identified by CAS registry number 923950-08-7, which provides the following chemical name: 7-37-Glucagon-like peptide I [8-glycine,22-glutamic acid,36-glycine] (synthetic human) fusion protein with peptide (synthetic 16-amino acid linker) fusion protein with immunoglobulin G4 (synthetic human Fc fragment), dimer. Each monomer of dulaglutide has the amino acid sequence set forth in SEQ ID NO:1:

HGEGTFTSDVSSYLEEQAAKEFIAWLVKGGGGGGGSGGGGSGGGGSAESKYGPPCPPCPA
PEAAGGPSVFLFPPKPKDTLMISRTPEVTCVVVDVSQEDPEVQFNWYVDGVEVHNAKTKP
REEQFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSRLT
VDKSRWQEGNVFSCSVMHEALHNHYTQKSLSLSLG

(SEQ ID NO:1).

The two monomers are attached by disulfide bonds between the cysteine residues at positions 55 and 58 to form the dimer. Dulaglutide's structure, function, production and use in treating T2DM is described in more detail in U.S. 7,452,966 and U.S. Patent Application Publication No. US20100196405. When used herein, the term "dulaglutide" refers to any GLP-1R agonist protein dimer of two monomers having the amino acid sequence of SEQ ID NO: 1, including any protein that is the subject of a regulatory submission seeking approval of a GLP-1R agonist product which relies in whole or part upon data submitted to a regulatory agency by Eli Lilly and Company relating to dulaglutide, regardless of whether the party seeking approval of said protein actually identifies the protein as dulaglutide or uses some other term.

Dulaglutide agonizes the GLP-1 receptor resulting in stimulation of insulin synthesis and secretion, and has been shown to provide improved glycemic control in T2DM patients. As described herein, however, the CKD-related improvements provided by the methods of the present invention - including in particular decreases in the rate of loss of eGFR - are not entirely dependent on any such improvements in glycemic control associated with GLP-1R mediated stimulation of insulin synthesis and secretion. In other words, while the known glycemic control benefits of dulaglutide in patients with T2DM may contribute to some CKD-related improvements in such patients, the CKD-related benefits provided by the present invention extend above and beyond any such improvements which may be attendant to improvements in glycemic control in T2DM patients. Thus, the benefits of the methods of the present invention in the treatment of CKD may be available for use in the treatment of patients for whom dulaglutide is not currently indicated for the purpose of providing improved glycemic control, such as patients with Type 1 diabetes mellitus (T1DM), or patients whose CKD is due to some cause other than diabetes, e.g., hypertension.

The population of patients characterized as having CKD includes patients across a broad spectrum of degrees of kidney function. That spectrum is commonly segmented into stages which are predominantly defined by ranges of eGFR. The National Kidney Foundation, sometimes referred to as KDOQI, publishes CKD treatment guidelines, which define 5 stages of CKD as follows in Table 1:

**Table 1.**

| Stage | Description | eGFR (mL/min/1.73 m²) |
|---|---|---|
| 1 | Kidney damage with normal or increased eGFR | ≥90 |
| 2 | Kidney damage with mild decrease in eGFR | 60-89 |
| 3 | Moderate decrease in eGFR | 30-59 |
| 4 | Severe decrease in eGFR | 15-29 |
| 5 | Kidney failure | <15 (or dialysis) |

Stage 3 CKD is further segmented by KDOQI into stages 3A and 3B, which are defined by eGFR ranges of 45 to 59 and 30 to 44, respectively.

As indicated above, stage 2 CKD requires kidney damage, which is defined in the KDOQI guidelines as pathologic abnormalities or markers of damage, including abnormalities in blood or urine tests or imaging studies. One measure that indicates the existence of kidney damage is the presence of albumin in the urine, or albuminuria. The severity of albuminuria is commonly categorized by the ratio of urine albumin (mg/dL) to urine creatinine (g/dL) (UACR). Albuminuria may be classified as microalbuminuria, when UACR is between about 30 and about 300 mg / g, or macroalbuminuria, when UACR is above 300 mg / g.

Other organizations do not use stage definitions for CKD patients, but instead define patients by their eGFR as follows: normal > 80, mild 50-80, moderate 30-50 and severe < 30.

When used herein, CKD "stage 2" refers to eGFR between 60-89 mL/min/1.73m² and kidney damage, "stage 3" refers to eGFR between 30-59 mL/min/1.73m², and the "stage 4" refers to eGFR between 15-29. In addition, CKD "stage 3A" refers to patients with eGFR between 45-59 and "stage 3B" refers to patients with eGFR between 30-44.

Current standard of care for patients with CKD includes ACE inhibitors and ARBs. Known ACE inhibitors include zofenopril, perindopril, trandolapril, captopril, enalapril, lisinopril, and ramipril. Known ARBs include valsartan, telmisartan, losartan, irbesartan, azilsartan, olmesartan, candesartan, eprosartan and fimasartan.

Such therapies are unable to stop progression of the disease, however, and CKD patients will continue to experience ongoing decline in kidney function as measured by loss of eGFR, as determined according to the CKD-EPI creatinine equation. *See*, *e*.*g*., Levey AS and Stevens LA, Estimating GFR using the CKD Epidemiology Collaboration (CKD-EPI) creatinine equation: more accurate GFR estimates, lower CKD prevalence estimates, and better risk predictions, AM. J. KIDNEY Dis. 2010;55(4):622-627.

For patients in stage 3 and 4, typical declines of eGFR are in the range of approximately 1-3 mL/min/1.73m² per year, even during treatment with standard of care. Once eGFR declines to consistently below 15 mL/min/1.73m², renal replacement therapy, such as dialysis or kidney transplant, is required. Thus, any treatment method capable of quickly attenuating the rate of eGFR decline, such as the methods described herein, may provide significant benefits to CKD patients who have not yet had to resort to renal replacement therapy.

The present invention is directed towards treatment of CKD in the latter stages of disease progression, but prior to kidney failure or renal replacement therapy, namely CKD stages 2, 3 or 4. In certain embodiments, the patient has stage 3 CKD. In other embodiments the patient has stage 3A or stage 3B CKD. In other embodiments the patient has stage 4 CKD. In certain embodiments the patient has a eGFR below 60 mL/min/1.73m² and/or eGFR between 60 and 89 mL/min/1.73m² with UACR above 30 mg/g. In other embodiments the patient has eGFR between 60 and 75 with UACR above 30 mg/g. In other embodiments, the patient has eGFR below 60 mL/min/1.73m², below 45 mL/min/1.73m² or below 30 mL/min/1.73m². In other embodiments the patient has eGFR between 15-75 mL/min/1.73m², 15-60 mL/min/1.73m², 30-60 mL/min/1.73m², 45-60 mL/min/1.73m², 15-30 mL/min/1.73m², 15-45 mL/min/1.73m² or 30-45 mL/min/1.73m². In certain embodiments the patient has UACR between 30-300 mg / g. In certain embodiments the patient has UACR ≥ 300 mg / g.

When used herein, the terms "treatment," "treat," "treating," and the like, are meant to include slowing or attenuating the progression of a disease or disorder. These terms also include alleviating, ameliorating, attenuating, eliminating, or reducing one or more symptoms of a disorder or condition, even if the disorder or condition is not actually eliminated and even if progression of the disorder or condition is not itself slowed or reversed.

A "patient" refers to a mammal, preferably a human with a disease, disorder or condition that would benefit from treatment for CKD.

The term "hazard ratio" refers to a measure of the relative rate of progression to an endpoint as compared to a control group. A hazard ratio value of 1 for example would indicate that the relative risks of the test and control groups in progressing to an endpoint are the same. A 15% reduction in the hazard ratio would indicate that the risk of the test group in progressing to an endpoint is 15% less than the risk that the control group progresses to an endpoint. In the context of the present invention, the comparator control group would refer to patients being treated with the standard of care, e.g., as described in the National Kidney Foundation KDOQI Clinical Practice Guidelines, and the endpoints could include a 40-50% reduction in eGFR, progression to ESRD, or death due to renal or cardiovascular causes. In certain embodiments, the methods of the present invention result in at least a 15% reduced hazard ratio. In certain embodiments, the methods of the present invention result in at least a 20% reduced hazard ratio. In certain embodiments, the methods of the present invention result in at hazard ratio reduced by 15-30%.

"HbA1c" refers to glycated hemoglobin levels, which develop when hemoglobin joins with glucose in the blood. HbA1c levels are a commonly used measure of glycemic control in patients with diabetes, with decreased HbA1c levels generally indicating improved glycemic control. In the context of the methods of the present invention, although dulaglutide is known to have benefits for glycemic control in T2DM patients, the CKD-related benefits described herein are not entirely dependent on such benefits in glycemic control.

In certain embodiments, the methods of the present invention result in a decrease in the rate of loss of eGFR. In certain embodiments, the methods of the present invention prolong the time to a 40% loss of eGFR. In certain embodiments, the methods of the present invention prolong the time to a 50% loss of eGFR. In certain embodiments, the methods of the present invention prolong the time to a 40-50% loss of eGFR.

In certain embodiments, the methods of the present invention prolong the time to the first to occur of any of renal death, renal replacement therapy and/or cardiovascular death in such patients. In certain embodiments, the methods of the present invention prolong the time to at least one of death due to renal causes, renal replacement therapy and/or cardiovascular death in such patients. In certain embodiments, the methods of the present invention result in a decrease in albuminuria.

When used herein the terms "decrease," "decreasing," "decreases," "prolong," "prolongs," "prolonging," "attenuate," "attenuates," "attenuating" and the like, refers to the effects of the methods of the present invention as compared to the effects of treatment with standard of care.

The methods of treatment and uses described herein may be provided in simultaneous or sequential combination with a standard of care. When used herein, the term "standard of care" refers to the maximum tolerated dose of ACE inhibitors and ARBs, and adequate treatment of blood pressure, lipids, and HbA1c to the local guidelines.

"Effective amount" means the amount of dulaglutide for the methods and uses of the present invention or pharmaceutical composition comprising dulaglutide for the methods and uses of the present invention that will elicit the biological or medical response of or desired therapeutic effect on a tissue, system, animal, mammal or human that is being sought by the researcher, medical doctor, or other clinician. An effective amount of dulaglutide may vary according to factors such as the disease state, age, sex, and weight of the individual, and the ability of dulaglutide to elicit a desired response in the individual. An effective amount is also one in which any toxic or detrimental effect is outweighed by the therapeutically beneficial effects.

Additional embodiments of the present invention are described below:
1. A method of treating CKD in a patient, comprising:
   a) identifying a patient having either:
      i) eGFR between 15-59 mL/min/1.73 m²; or
      ii) UACR greater than 30 mg / g and eGFR between 60-89 mL/min/1.73 m²;
   b) administering said patient an effective amount of dulaglutide once a week; and
   c) continuing said once a week administration for at least 6 months.
2. The method of the above embodiment wherein said administration results in attenuation in the progression of the patient's CKD.
3. The method of the above embodiment wherein said attenuation in the progression of the patient's CKD is not entirely dependent on improvements to the patient's glycemic control.
4. A method of decreasing the rate of loss of eGFR in a patient having CKD, comprising:
   a) identifying a patient having either:
      i) eGFR between 15-59 mL/min/1.73 m²; or
      ii) UACR greater than 30 mg / g and eGFR between 60-89 mL/min/1.73 m²;
   b) administering said patient an effective amount of dulaglutide once a week; and continuing said once a week administration for at least 6 months.
5. The method of the above embodiment wherein the decreased rate of loss of eGFR is not entirely dependent on improvements to the patient's glycemic control.
6. Dulaglutide for use in treating CKD in a patient, wherein the patient has either:
   a) eGFR between 15-59 mL/min/1.73 m²; or
   b) UACR greater than 30 mg / g and eGFR between 60-89 mL/min/1.73 m²; wherein an effective amount of dulaglutide is administered once a week for at least 6 months,
7. The use of the above embodiment wherein said administration results in attenuation in the progression of the patient's CKD.
8. The use of any of the above embodiments wherein said attenuation in the progression of the patient's CKD is not entirely dependent on improvements to the patient's glycemic control.
9. Dulaglutide for use in decreasing the rate of loss of eGFR in a patient having CKD, wherein the patient has either:
   a) eGFR between 15-59 mL/min/1.73 m²; or
   b) UACR greater than 30 mg / g and eGFR between 60-89 mL/min/1.73 m²; and wherein an effective amount of dulaglutide is administered once a week for at least 6 months.
10. The use of the above embodiment wherein the decreased rate of loss of eGFR is not entirely dependent on improvements to the patient's glycemic control.
11. The method of any of the above embodiments wherein the patient's CKD is not caused by T2DM.
12. The method of any of the above embodiments wherein the patient's CKD is caused by T2DM.
13. The method of any of the above embodiments wherein the patient has eGFR between 15-59 mL/min/1.73 m².
14. The method of any of the above embodiments wherein the patient has eGFR between 15-29 mL/min/1.73 m².
15. The method of any of the above embodiments wherein the patient has eGFR between 30-59 mL/min/1.73 m².
16. The method of any of the above embodiments wherein the patient has eGFR between 30-44 mL/min/1.73 m².
17. The method of any of the above embodiments wherein the patient has eGFR between 45-59 mL/min/1.73 m².
18. The method of any of the above embodiments wherein the patient has eGFR between 15-44 mL/min/1.73 m².
19. The method of any of the above embodiments wherein the patient has a UACR is ≥ 30 mg / g and an eGFR between 60-75 mL/min/1.73 m².
20. The method of any of the above embodiments wherein the patient's UACR is between 30-300 mg/g.
21. The method of any of the above embodiments wherein the patient's UACR is ≥ 300 mg/g.
22. The method of any of the above embodiments wherein said administration is continued for at least 1 year.
23. The method of any of the above embodiments wherein said administration is continued for at least 2 years.
24. The method of any of the above embodiments wherein said administration is continued for at least 3 years.
25. The method of any of the above embodiments wherein said administration is continued for at least 4 years.
26. The method of any of the above embodiments wherein said administration is continued for at least 5 years.
27. The method of any of the above embodiments wherein said administration is continued for at least 10 years.
28. The method of any of the above embodiments wherein the effective amount of dulaglutide is between 0.75-4.5 mg.
29. The method of any of the above embodiments wherein the effective amount of dulaglutide is selected from the group consisting of 0.75 mg, 1.5 mg, 3.0 mg and 4.5 mg.
30. The method of any of the above embodiments wherein the effective amount of dulaglutide is 0.75 mg.
31. The method of any of the above embodiments wherein the effective amount of dulaglutide is 1.5 mg.
32. The method of any of the above embodiments wherein the effective amount of dulaglutide is 3.0 mg.
33. The method of any of the above embodiments wherein the effective amount of dulaglutide is 4.5 mg.
34. The method of any of the above embodiments wherein said attenuation in progression of the patient's CKD is reflected in an increase in time to one or more of: a 40% decrease in eGFR; progression to ESRD; renal death; and/or cardiovascular death.
35. The method of any of the above embodiments wherein said attenuation in progression of the patient's CKD is in reflected in an increase in time to one or more of: a 50% decrease in eGFR; progression to ESRD; renal death; and/or cardiovascular death.
36. The method of any of the above embodiments wherein said attenuation in progression of the patient's CKD is in reflected in an increase in time to a doubling of serum creatinine.
37. The method of any of the above embodiments further comprising administering an ACE inhibitor and/or an ARB.
38. The method of any of the above embodiments further comprising administering a rapid acting insulin analog selected from the group consisting of insulin lispro and insulin aspart.
39. The method of any of the above embodiments further comprising administering a rapid acting insulin analog selected from the group consisting of insulin lispro and insulin aspart and wherein the rapid acting insulin analog is titrated to target pre-prandial glucose levels of 120-180 mg/dL (6.7-10 mmol/L).
40. The use of any of the above embodiments wherein the patient's CKD is not caused by T2DM.
41. The use of either of any of the above embodiments wherein the patient's CKD is caused by T2DM.
42. The use of any of the above embodiments wherein the patient has eGFR between 15-59 mL/min/1.73 m².
43. The use of any of the above embodiments wherein the patient has eGFR between 15-29 mL/min/1.73 m².
44. The use of any of the above embodiments wherein the patient has eGFR between 30-59 mL/min/1.73 m².
45. The use of any of the above embodiments wherein the patient has eGFR between 30-44 mL/min/1.73 m².
46. The use of any of the above embodiments wherein the patient has eGFR between 45-59 mL/min/1.73 m².
47. The use of any of the above embodiments wherein the patient has eGFR between 15-44 mL/min/1.73 m².
48. The use of any of the above embodiments wherein the patient has kidney damage eGFR between 60-75 mL/min/1.73 m².
49. The use of any of the above embodiments wherein the patient has a UACR between 30-300 mg/g.
50. The use of any of the above embodiments wherein the patient has a UACR greater than 300 mg/g.
51. The use of any of the above embodiments wherein said administration is continued for at least 1 year.
52. The use of any of the above embodiments wherein the effective amount of dulaglutide is between 0.75-4.5 mg.
53. The use of any of the above embodiments wherein the effective amount of dulaglutide is selected from the group consisting of 0.75 mg, 1.5 mg, 3.0 mg and 4.5 mg.
54. The use of any of the above embodiments wherein said attenuation in progression of the patient's CKD is reflected in an increase in time to one or more of: a 40% decrease in eGFR; progression to ESRD; renal death; and/or cardiovascular death.
55. The use of any of the above embodiments wherein said attenuation in progression of the patient's CKD is in reflected in an increase in time to one or more of: a 50% decrease in eGFR; progression to ESRD; renal death; and/or cardiovascular death.
56. Any of the embodiments wherein the hazard ratio is reduced by at least 15%.
57. Any of the above embodiments wherein the hazard ratio is reduced by at least 20%.
58. Any of the above embodiments wherein the hazard ratio is reduced by 15-30%.

The invention is further illustrated by the following examples, which are not to be construed as limiting.

### EXAMPLES

In a phase 3 clinical study, once weekly dulaglutide in doses of 0.75 mg or 1.5 mg is compared to daily titrated insulin glargine, each combined with insulin lispro provided for postprandial glucose control, in patients with moderate and severe CKD and T2DM. The study is designed as a multicenter, parallel-arm, randomized, 52-week treatment study assessing the efficacy and safety of dulaglutide compared to insulin glargine. The primary objective is to determine whether dulaglutide is noninferior to insulin glargine with respect to HbA1c in this patient population at 26 weeks, but secondary objectives were also designed to determine effects on eGFR and albuminuria.

Participants are randomized (1:1:1) to once weekly dulaglutide 1.5 mg (N=192), once weekly dulaglutide 0.75 mg (N=190) or titrated insulin glargine (N=194). Stratification factors included time of enrollment, macroalbuminuria, geographic region, and CKD stage.

Baseline characteristics are similar between treatment groups, including for CKD related characteristics, as indicated in Table 2 below:

**Table 2. Data presented as mean ± SD or n (%), Abbreviations: DU=dulaglutide; mITT=modified intent-to-treat population, which includes all randomized patients who receive at least 1 dose of randomized treatment (dulaglutide or insulin glargine) and who have at least 1 post-randomization HbA1c value, classified according to their assigned treatment.**

| Baseline Characteristics (mITT population) | DU 1.5 mg N=183 | DU 0.75 mg N=180 | Insulin Glargine N=186 |
|---|---|---|---|
| Duration of diabetes, years | 17.7 ± 8.8 | 18.0 ± 8.9 | 18.6 ± 8.8 |
| Duration of CKD stage 3 or higher, years | 4.2 ± 5.7 | 4.1 ± 5.0 | 3.5 ± 4.0 |
| HbA1c, % | 8.6 ± 0.9 | 8.6 ± 1.1 | 8.6 ± 1.0 |
| eGFR (CKD-EPI creatinine equation), ml/min/1.73m² | 38.0 ± 13.3 | 38.4 ± 12.3 | 38.5 ± 13.0 |
| 60 ≤ Baseline eGFR <90 | 8 (4.4) | 6 (3.3) | 13 (7.0) |
| 45 ≤ Baseline eGFR <60 | 51 (27.9) | 51 (28.3) | 50 (26.9) |
| 30 ≤ Baseline eGFR <45 | 70 (38.3) | 72 (40.0) | 64 (34.4) |
| 15 ≤ Baseline eGFR <30 | 52 (28.4) | 51 (28.3) | 58 (31.2) |
| Baseline eGFR <15 | 2 (1.1) | 0 (0.0) | 1 (0.5) |
| UACR, mg/g | 756.5 ± 1294.7 | 839.0 ± 1383.6 | 891.6 ± 1501.3 |
| 30 ≤ UACR ≤ 300 (mg/g) | 73 (39.9) | 56 (31.3) | 55 (29.6) |
| UACR >300 (mg/g) | 80 (43.7) | 81 (45.3) | 84 (45.2) |

26 week A1c data are provided in Table 3 below:

**Table 3. Data are reported as LSM (SE) unless otherwise indicated. ^{†}, ^{††} multiplicity adjusted 1-sided p<0.001 for noninferiority versus insulin glargine with a 0.4% margin or 0.3% margin, respectively, and *2-sided p<0.001 change from baseline. Table Excluding Data after Rescue or Study Drug Discontinuation. Abbreviations: LSM=least squares mean; SE = standard error; pt=participant(s).**

| Primary Endpoint (26 week, mITT population) | DU 1.5 mg (N=183) | DU 0.75 mg (N=180) | Insulin Glargine (N=186) |
|---|---|---|---|
| HbA1c change (%) | -1.19 | -1.12 (0.12)^{†∗} | -1.13 (0.13)* |
| | (0.13)^{††∗} | | |
| Percentage of pt with A1c <7% | 37.5 | 31.7 | 34.6 |
| Percentage of pt with A1c <8% | 78.3 | 72.6 | 75.3 |

The data support that dulaglutide produced comparable glycemic control as compared to insulin glargine.

26-week data on eGFR and albuminuria in the overall study participants and by UACR > 300 mg/g and UACR ≤ 300 mg/g are provided in Table 4 below.

**Table 4. 26-week data on eGFR are presented as change from baseline LSM (95% CI), and data on UACR are presented as percent change from baseline LSM (95% CI) as obtained via log-transformed analysis; safety population, which includes all patients (regardless of missing post-randomization HbA1c data), classified according to the treatment actually received. *2-sided p < 0.05 and **2-sided p < 0.001 change from baseline, #2-sided p<0.05 versus insulin glargine. Abbreviations: CI=confidence interval.**

| Treatment arm | All Participants | | Participants with UACR > 300 mg/g (n=258) | | Participants with UACR ≤ 300 mg/g (n=317) | |
|---|---|---|---|---|---|---|
| | Δ eGFR, mL / min / 1. 73m² (n=576) | %Δ UACR (n=575) | Δ eGFR, mL / min / 1. 73m² | %Δ UACR | Δ eGFR, mL / min / 1. 73m² | %Δ UACR |
| DU 1.5 mg | -0.1# (-1.2, 1.0) | -27.7** (-38.7, -14.8) | -1.9*# (-3.5, -0.4) | -43.1**# (-54.7, -28.6) | 0.3 (-1.0, 1.7) | -0.4 (-19.2, 22.8) |
| DU 0.75 mg | -0.4# (-1.4, 0.7) | -26.7** (-37.9, -13.5) | -2.6**# (-4.2, -1.1) | -25.3 * (-40.2, -6.8) | 0.3 (-1.0, 1.7) | -18.0 (-33.6, 1.3) |
| Glargine | -1.9** (-3.0, -0.9) | -16.4* (-29.0, -1.5) | -4.8** (-6.3, -3.4) | -14.3 (-30.9, 6.3) | -0.7 (-2.0, 0.7) | -5.7 (-23.2, 15.8) |

After 26 weeks eGFR for the all participants group, and participants with UACR > 300 mg/g group, decreased significantly with insulin glargine, indicating an expected progression of CKD. eGFR surprisingly remained stable with dulaglutide in the all participants group, and decreased significantly less than observed for insulin glargine in the UACR >300 mg/g group. Participants in the UACR > 300 mg/g group receiving dulaglutide 1.5 mg also had greater reductions in UACR compared to insulin glargine.

52 week A1c data are provided in Table 5 below:

**Table 5. Data are reported as LSM (SE) unless otherwise indicated. ^{†}, ^{††} multiplicity adjusted 1-sided p<0.001 for noninferiority versus insulin glargine with a 0.4% margin or 0.3% margin, respectively, and *2-sided p<0.001 change from baseline. Excluding Data after Rescue or Study Drug Discontinuation.**

| Primary Endpoint (52 week, mITT population) | DU 1.5 mg (N=183) | DU 0.75 mg (N=180) | Insulin Glargine (N=186) |
|---|---|---|---|
| A1c change, % | -1.10 (0.13)^{††∗} | -1.10 (0.12)^{†∗} | -1.00 (0.12)* |
| Percentage of pt with A1c <7% | 32.9 | 33.5 | 29.1 |
| Percentage of pt with A1c <8% | 69.1 | 69.5 | 70.3 |

52-week data on eGFR and albuminuria in the overall study participants and by UACR > 300 mg/g and UACR ≤ 300 mg/g are provided in Table 6 below.

**Table 6. 52-week data on eGFR are presented as change from baseline LSM (95% CI), and data on UACR are presented as percent change from baseline LSM (95% CI) as obtained via log-transformed analysis; safety population, which includes all patients (regardless of missing post-randomization A1c data), classified according to the treatment actually received. *2-sided p < 0.05 and **2-sided p < 0.001 change from baseline, #2-sided p<0.05 versus insulin glargine.**

| Treatment arm | All Participants (N=576) | | Participants with UACR > 300 mg/g (n=258) | | Participants with UACR ≤ 300 mg/g (n=317) | |
|---|---|---|---|---|---|---|
| | Δ eGFR, mL / min / 1.73m² | %Δ UACR | Δ eGFR, mL / min / 1.73m² | %Δ UACR | Δ eGFR, mL / min / 1.73m² | %Δ UACR |
| DU 1.5 mg | -1.1 (-2.4, 0.2) | -22.5* (-35.1, -7.5) | -3.4**# (-5.4, -1.4) | -29.0*# (-43.0, -11.5) | -1.4 (-2.0, 1.3) | -3.4 (-24.0, 22.8) |
| DU 0.75 mg | -1.5* (-2.8,-0.2) | -20.1* (-33.1, -4.6) | -5.2** (-7.1, -3.2) | -12.3 (-29.0, 8.5) | 0.2 (-1.4, 1.9) | -15.3 (-33.6, 8.0) |
| Insulin Glargine | -2.9^{∗∗} (-4.2,-1.6) | -13.0 (-27.1, 3.9) | -6.3 * * (-8.2, -4.4) | 0.1 (-18.8, 23.4) | -1.3 (-2.9, 0.4) | -9.9 (-29.0, 14.4) |

The 52-week data in Tables 5 and 6 show that dulaglutide continues to provide comparable glycemic control to insulin glargine out to 52 weeks, but that the attenuated rate of eGFR decline and reductions in UACR observed compared to insulin glargine after 26 weeks are maintained, particularly for participants with UACR > 300 mg/g receiving 1.5 mg dulaglutide.

## Claims

1. Dulaglutide for use in treating CKD in a patient, wherein the patient has either:
a) eGFR between 15-59 mL/min/1.73 m²; or
b) UACR greater than 30 mg / g and eGFR between 60-89 mL/min/1.73 m²; wherein an effective amount of dulaglutide is administered once a week for at least 6 months; and wherein said administration results in attenuation in the progression of the patient's CKD.

2. Dulaglutide for use according to claim 1 wherein said attenuation in the progression of the patient's CKD is not entirely dependent on improvements to the patient's glycemic control.

3. Dulaglutide for use in decreasing the rate of loss of eGFR in a patient having CKD, wherein the patient has either:
a) eGFR between 15-59 mL/min/1.73 m²; or
b) UACR greater than 30 mg / g and eGFR between 60-89 mL/min/1.73 m²; and wherein an effective amount of dulaglutide is administered once a week for at least 6 months.

4. Dulaglutide for use according to claim 3 wherein the decreased rate of loss of eGFR is not entirely dependent on improvements to the patient's glycemic control.

5. Dulaglutide for use according to any one of claims 1 to 4 wherein the patient's CKD is not caused by T2DM.

6. Dulaglutide for use according to either of any one of claims 1 to 4 wherein the patient's CKD is caused by T2DM.

7. Dulaglutide for use according to any one of claims 1 to 4 wherein the patient has eGFR between 15-59 mL/min/1.73 m².

8. Dulaglutide for use according to any one of claims 1 to 4 wherein the patient has eGFR between 15-29 mL/min/1.73 m².

9. Dulaglutide for use according to any one of claims 1 to 4 wherein the patient has eGFR between 30-59 mL/min/1.73 m².

10. Dulaglutide for use according to any one of claims 1 to 4 wherein the patient has eGFR between 30-44 mL/min/1.73 m².

11. Dulaglutide for use according to any one of claims 1 to 4 wherein the patient has eGFR between 45-59 mL/min/1.73 m².

12. Dulaglutide for use according to any one of claims 1 to 4 wherein the patient has eGFR between 15-44 mL/min/1.73 m².

13. Dulaglutide for use according to any one of claims 1 to 4 wherein the patient has kidney damage eGFR between 60-75 mL/min/1.73 m².

14. Dulaglutide for use according to any one of claims 1 to 4 wherein the patient has a UACR between 30-300 mg/g.

15. Dulaglutide for use according to any of claims 1 to 4 wherein the patient has a UACR greater than 300 mg/g.

16. Dulaglutide for use according to any one of claims 13 to 15, wherein said administration is continued for at least 1 year.

17. Dulaglutide for use according to any of claims 1 to 16, wherein the effective amount of dulaglutide is between 0.75-4.5 mg.

18. Dulaglutide for use according to any one of claims 1 to 16, wherein the effective amount of dulaglutide is selected from the group consisting of 0.75 mg, 1.5 mg, 3.0 mg and 4.5 mg.

19. Dulaglutide for use according to any one of claims 1 to 16, wherein said attenuation in progression of the patient's CKD is reflected in an increase in time to one or more of: a 40% decrease in eGFR; progression to ESRD; renal death; and/or cardiovascular death.

20. Dulaglutide for use according to any one of claims 1 to 16, wherein said attenuation in progression of the patient's CKD is in reflected in an increase in time to one or more of: a 50% decrease in eGFR; progression to ESRD; renal death; and/or cardiovascular death.
